# EUROPEAN PATENT SPECIFICATION

(11) **EP 0 550 183 B1**
(45) Date of publication and mention of the grant of the patent: **13.09.1995**
(21) Application number: 92311286.6
(22) Date of filing: 10.12.1992
(51) Int. Cl.: C07C 31/12, C07C 29/88

(54) **Purification of tertiary butyl alcohol**
Reinigung von Tertiärbutylalkohol
Purification de l'alcool tertio-butylique

(30) Priority: 16.12.1991 US 807360
(43) Date of publication of application: 07.07.1993
(73) Proprietor: TEXACO CHEMICAL INC., Houston, Texas 77056 (US)
(72) Inventor: Smith, William Alan, Houston, Texas 77068 (US); Tooloian, Roya, Houston, Texas 77058 (US)
(74) Representative: Green, Mark Charles

(56) References cited:
- FR-A- 1 497 444
- GB-A- 687 380
- GB-A- 825 359
- GB-A- 1 443 106
- US-A- 3 462 500
- US-A- 3 576 891
- 'ION EXCHANGERS' 1991 , WALTER DE GRUYTER , BERLIN
- CHEMICAL ABSTRACTS, vol. 105, no. 8, 25 August 1986, Columbus, Ohio, US; abstract no. 62646k, H. M. LLANEZA COALLA 'Hydrolysis of isoamyl acetate in a continuous flow catalyzed by an anion-exchange resin.' page 118 ;column 1 ;

## Description

This invention relates to the purification of tertiary butyl alcohol (TBA). More particularly, this invention relates to a process for removing contaminating quantities of tertiary butyl formate from impure tertiary butyl alcohol.

In accordance with the present invention, the impure tertiary butyl alcohol distillation fraction is brought into contact with styrene-divinyl benzene anionic ion exchange resin in a treating zone for a period of time sufficient to substantially completely convert the tertiary butyl formate to formic acid and tertiary butyl alcohol. A partially purified substantially tertiary butyl formate-free tertiary butyl alcohol fraction is recovered from the treating zone.

It is known to react isobutane with oxygen, either thermally or catalytically, to form peroxidation reaction product wherein the principal peroxide that is formed is tertiary butyl hydroperoxide. It is also known to prepare tertiary butyl alcohol directly from tertiary butyl hydroperoxide or to react the tertiary butyl hydroperoxide with an olefin such as propylene in the presence of a soluble molybdenum catalyst to form an olefin epoxide such as propylene oxide and tertiary butyl alcohol.

The epoxidation reaction mixture that is formed when propylene is reacted with tertiary butyl hydroperoxide in solution with tertiary butyl alcohol in the presence of a soluble molybdenum epoxidation catalyst will normally comprise unreacted propylene, propylene oxide, unreacted tertiary butyl hydroperoxide, tertiary butyl alcohol, a soluble molybdenum catalyst and impurities including tertiary butyl formate. The reaction mixture is normally separated by distillation into a plurality of distillation fractions including a recycle propylene fraction, a propylene oxide fraction, a tertiary butyl alcohol fraction, and a heavy liquid distillation fraction containing tertiary butyl alcohol, unreacted tertiary butyl hydroperoxide, heavy impurities and substantially all of the dissolved molybdenum catalyst.

Tertiary butyl formate will also be present as a contaminant when tertiary butyl alcohol is formed directly from tertiary butyl hydroperoxide. Tertiary butyl formate is very difficult to remove from tertiary butyl alcohol by conventional means, e.g. distillation.

It is known to react a hydroperoxide feedstock such as tertiary butyl hydroperoxide with propylene in solvent solution in the presence of an epoxidation catalyst in order to provide a reaction product comprising unreacted feed components, propylene oxide, t-butyl alcohol, a solvent (which may also be t-butyl alcohol), and impurities (see, for example, Kollar U. S. Patent No. 3,350,422, Kollar U. S. Patent No. 3,351,635 and Sorgenti U. S. Patent No. 3,666,777.

It is also known to separate the reaction product by distillation in order to obtain a plurality of fractions including, for example, a propylene recycle fraction, a propylene oxide product fraction, a tertiary butyl alcohol fraction.

It is known to remove methyl formate from propylene oxide by treating the impure propylene oxide with an aqueous base, as shown, for example, by Mitchell et al. U. S. Patent No. 2,550,847, Lichtenwalter et al. U. S. Patent No. 3,477,919 and Sanderson et al. U. S. Patent No. 4,691,035.

It is also known to remove methyl formate from impure propylene oxide by extractive distillation as shown, for example, by Kageyama et al. U. S. Patent No. 3,838,020.

Robeson et al. U. S. Patent No. 2,622,060 discloses the removal of methyl formate by extractive distillation employing an aqueous solution of an alkaline compound as the extractant.

Binning et al. U. S. Patent No. 3,350,417 discloses a method for the production and recovery of propylene oxide by plural stage distillation and caustic addition wherein a methyl formate is removed.

For example, Mitchell et al. U. S. Patent No. 2,550,847 is directed to a process for separating purified propylene oxide from a crude propylene oxide product contaminated with acetaldehyde, methyl formate, methanol, etc., by treating the crude mixture with an aqueous base followed by recovery of the purified propylene oxide by any suitable means such as by decantation. Mitchell et al. reported a recovery of a product containing 78 to 82 wt.% of propylene oxide which, they stated, could be increased in purity to about 95 to 99% by fractional distillation.

US-3576891 (Rosenthal) describes a method for the removal of impurities, particularly formic acid and formates, from tertiary butyl alcohol. The citation discloses the use of a supported alkaline metal hydroxide catalyst for the removal of the impurities. The process is carried out in the vapour phase.

Ion Exchangers, 1991, pages 1011-1012, describes that esters may be hydrolysed using cation exchange resins. Furthermore, esters of lower-molecular weight acids can be hydrolysed by anion exchangers.

Chemical Abstracts, Vol. 105, Abstract 6-646 K, describes the hydrolysis of isoamyl acetate in a continuous flow catalyzed by an anion-exchange resin.

In accordance with the present invention, an impure tertiary butyl alcohol fraction, such as an impure tertiary butyl alcohol distillation fraction, is obtained by the distillation of the reaction mixture formed when tertiary butyl hydroperoxide is directly converted to tertiary butyl alcohol or propylene is reacted with tertiary butyl hydroperoxide in solution in tertiary butyl alcohol in the presence of a soluble molybdenum catalyst. The impure tertiary butyl alcohol distillation fraction will typically contain from about 50 to about 5,000 ppm of tertiary butyl formate.

In accordance with the present invention, the impure tertiary butyl alcohol contaminated with tertiary butyl formate is brought into contact with a styrene-divinyl benzene anionic ion exchange resin in a treating zone and contacted therein for a period of time sufficient to substantially completely convert the tertiary butyl formate to tertiary butyl alcohol and formic acid. The formic acid will remain adsorbed on the basic ion exchange resin while the generated tertiary butyl alcohol will pass through the treating zone with the tertiary butyl alcohol which was initially present in the initial charge.

When tertiary butyl alcohol is prepared directly from tertiary butyl hydroperoxide or by reaction with an olefin such as propylene in the presence of a soluble molybdenum catalyst to form an olefin epoxide such as propylene oxide and tertiary butyl alcohol a reaction mixture is formed that contains both tertiary butyl alcohol and tertiary butyl formate. When tertiary butyl alcohol is recovered from the reaction mixture, at least a portion of the tertiary butyl formate will frequently be present in the recovered tertiary butyl alcohol as a contaminant. The "recovered tertiary butyl alcohol" is frequently referred to herein as "impure tertiary butyl alcohol".

Although the impure tertiary butyl alcohol fraction obtained in this fashion will normally be composed of about 95 wt.% or more of tertiary butyl alcohol, oxygen-containing impurities such as tertiary butyl formate are contained therein and are removed only with difficulty.

It has been discovered in accordance with the present invention that when the impure tertiary butyl alcohol feedstock is fed to a treating zone containing a styrenedivinyl benzene anionic ion exchange resin the tertiary butyl formate can be removed therefrom without loss of tertiary butyl alcohol.

### BRIEF DESCRIPTION OF THE DRAWING

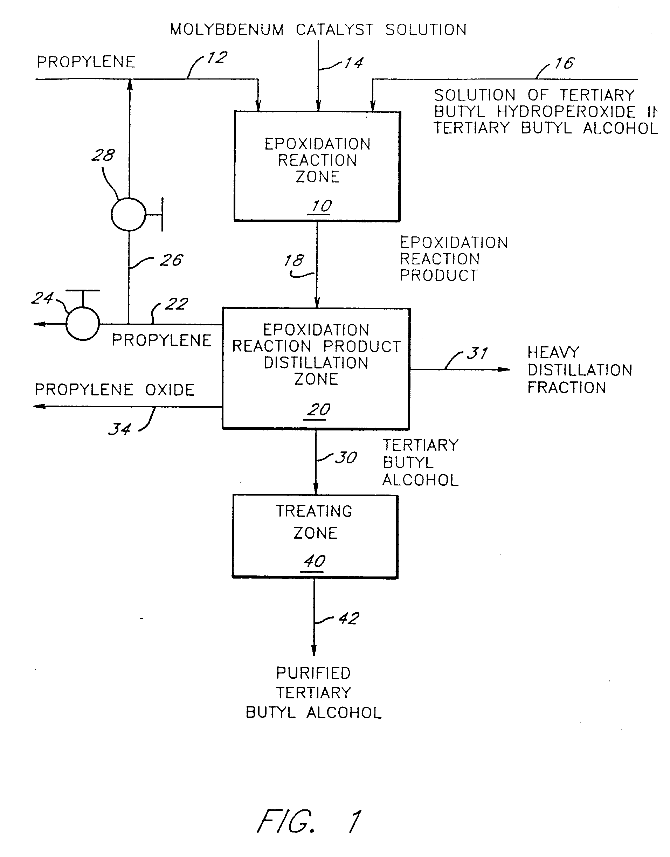

In the drawing, Fig. 1 is a schematic flow chart illustrating the manner in which propylene and tertiary butyl hydroperoxide are reacted in solution in tertiary butyl alcohol in the presence of a molybdenum catalyst to provide an epoxidation reaction product from which a tertiary butyl alcohol fraction is recovered by distillation and to the purification of the tertiary butyl alcohol distillation fraction by the removal of tertiary butyl formate therefrom.

With reference to the drawing, a preferred method will be described.

An epoxidation reaction zone 10 is provided and propylene is charged thereto by a line 12 together with a soluble molybdenum catalyst charged by a line 14. A solution of tertiary butyl hydroperoxide and tertiary butyl alcohol is charged by a line 16.

The epoxidation reaction is an epoxidation reaction of the type disclosed by Kollar U. S. Patent No. 3351635 as further elaborated upon, for example, in British patent specification No. 1,298,253 wherein propylene is reacted with tertiary butyl hydroperoxide under reaction conditions including a reaction temperature within the range of 82° to 149°C (180° to 300°F), a pressure of 2068 to 6895 kPa gauge pressure (gp) (300 to 1000 psig) and, more preferably, a temperature of 104° to 138°C (220° to 280°F) and a pressure of 3447 to 5516 kPa gp (500 to 800 psig).

The soluble molybdenum catalyst charged to the epoxidation reaction zone by the line 14 may be an epoxidation catalyst of the type known in the art such as those disclosed by the Kollar patent or the British patent or by Marquis et al. U. S. Patent No. 4,626,596, U. S. Patent No. 4,650,886, U. S. Patent No. 4,654,427, or U. S. Patent No. 4,758,681. The Marquis et al. patents are directed to molybdenum/alkanol complexes such as solutions of molybdenum compounds in ethylene glycol which contain a high concentration of molybdenum and are particularly useful as catalysts in the epoxidation reaction. Marquis et al. teach, for example, the epoxidation of propylene with tertiary butyl hydroperoxide with their catalyst under epoxidation conditions including a temperature of 50 to 180°C. and a use of propylene to tertiary butyl hydroperoxide molar ratios within the range of 0.9:1 to 3.0:1.

Suitably, the tertiary butyl hydroperoxide that is charged to the epoxidation reaction zone 10 by way of line 16 is a 40 to 75 wt.% solution of tertiary butyl hydroperoxide in tertiary butyl alcohol. The catalyst is charged to the epoxidation reaction zone 10 by the charge line 14 in an amount such as to provide from 50 to 1000 ppm of molybdenum, based on the total of the reactants charged and, more preferably, from 200 to 600 ppm. The reaction is preferably conducted at superatmospheric pressure such as a pressure of 2068 to 6895 kPa gp (300 to 1000 psig).

When the reaction is conducted on a continuous basis, as illustrated in the drawing, the feed materials are charged to the epoxidation reaction zone 10 through the lines 12, 14 and 16 at rates sufficient to maintain the desired concentration of reactants and an equivalent mass of epoxidation reaction mixture is withdrawn from the epoxidation reaction zone 10 by way of a discharge line 18. The reaction product discharged by the line 18 will normally comprise unreacted propylene, a minor amount of unreacted tertiary butyl hydroperoxide, propylene oxide, tertiary butyl alcohol, including tertiary butyl alcohol formed by the reaction of the tertiary butyl hydroperoxide with propylene, the molybdenum catalyst and impurities such as propane, propionaldehyde, acetone, methanol, isopropanol, water, acetaldehyde, methyl formate, tertiary butyl formate, acetic acid, formic acid, isobutyric acid, hydrocarbons containing 6 or more carbon atoms and high boiling residue components.

The reaction product 18 is charged to an epoxidation reaction product distillation zone 20 where it is separated by distillation into desired fractions in accordance with methods known to those skilled in the art. For example, the distillation sequence disclosed in British Patent No. 1,298,253 may be used.

One of the distillate products that is recovered in the zone 20 is a propylene fraction which is discharged by a line 22 controlled by a valve 24 and provided with a branch line 26 controlled by a valve 28 in order to permit the recycle of unreacted propylene to the epoxidation reaction zone 10 through the propylene charge line 12.

Another distillate fraction that is obtained is a propylene oxide product fraction which is discharged by the line 34.

Another product that is recovered from the epoxidation reaction product distillation zone 20 is a tertiary butyl alcohol distillate product discharged through line 30 which may be further purified in accordance with the present invention.

A heavy distillation fraction 31, usually a bottoms fraction, is also discharged from the epoxidation reaction product distillation zone 20.

The anion exchange resins to be used in accordance with the present invention are styrene-divinyl benzene anionic ion exchange resins. These consist essentially of a styrene polymer resin, such as a divinyl benzene cross-linked polystyrene matrix having 0.5 to 20% and preferably 4 to 16% of copolymerized divinyl benzene therein, bearing ionizable or functional base groups. These resins are manufactured and sold commercially under various trade names such as "Amberlite IRA400", "Amberlite IRA68" and "Amberlyst A-26".

The properties of basic ion exchange catalysts are also shown, for example, in a technical brochure entitled "Ion Exchange Catalysis and Matrix Effects" by Arpitochelli, published by Rohm & Haas in 1980 and by other technical bulletins and brochures published by Rohm & Haas including, for example, a technical bulletin entitled "Technical Bulletin Fluid Process Chemicals" published in 1986.

In the Rohm and Haas technical bulletin, their Gel Type Amberlite® styrene-divinyl benzene ion exchange resins are characterized as "Strongly Acidic Cation Exchangers", "Weakly Acidic Cation Exchangers", "Strongly basic Anion Exchangers", and "Weakly basic Anion Exchangers". The preferred basic ion exchange resins to be used in accordance with the present invention are of the type characterised in the Rohm and Haas bulletin as "Strongly basic Anion Exchangers".

The impure tertiary butyl alcohol distillation fraction is treated in treating zone 40 for a period of time sufficient to substantially completely convert the tertiary butyl formate to formic acid and additional tertiary butyl alcohol. For example, the fraction may be treated at a temperature within the range of about 0 to about 130°C. and passed through the treating zone 40 at a hourly weighted space velocity (WHSV) of 2 to 5 kg of impure tertiary butyl alcohol per kg of basic ion exchange resin per hour.

The thus-treated partially purified tertiary butyl alcohol is discharged from treating zone 40 by a line 42.

The treating zone 40 contains a bed of a suitable basic ion exchange resin such as Amberlite® IRA-400. For example, the treating conditions established in the treating zone may include, for example, a temperature of about 0 to about 130°C. The pressure is preferably sufficient to keep the reactants in liquid phase. The tertiary butyl alcohol may be passed through the bed of ion exchange resin at a weighted hourly space velocity of from 2 to 5 kg of impure tertiary butyl alcohol distillation fraction 30 per kg of basic ion exchange bed. As a consequence, substantially all of the tertiary butyl formate charged by way of the line 30 is converted to formic acid and additional tertiary butyl alcohol. The formic acid stays adsorbed to the basic resin matrix until the basic resin matrix is regenerated by a suitable regeneration method of the type known to those skilled in the art.

### EXAMPLES

### Example 1

In reaction run 1, an 80 mls feed solution containing 95.65 wt.% TBA and 0.139 wt.% TBF was mixed with 34.9 g of Amberlite® (IRA-400) catalyst in a batch system at room temperature. Samples were taken at periods of 30 minutes and 60 minutes to test the variation in concentration of TBA, TBF and the acid number. Using a GC analysis it was observed that the TBF concentration was reduced to .038 wt.% after 30 minutes and .009 wt.% after 60 minutes. An acid test showed the reduction of acid number from 4.20 mg KOH/g sample in the feed to .045 after 30 minutes and .015 after 60 minutes.

### Example 2

In reaction run 2 similar feed as Example 1 was used with concentration of 96.93 wt.% TBA and 1.05 wt.% TBF. 34.9 g of IRA-400 catalyst was used with similar manner as above. Using GC analysis TBF concentration was reduced to .011 wt.% after 30 minutes and .007 wt.% after 60 minutes.

### Example 3

In reaction run 3 similar feed and catalyst were used to verify TBF concentration reduction using IRA-400 catalyst. Using GC analysis, after 30 minutes the concentration of TBA changed from 96.93 wt.% to 97.52 wt.% where the TBF concentration changed from 1.05 wt.% to .382 wt.%. After 60 minutes the TBA concentration changed to 98.11 and TBF changed to .129 wt.% and after 90 minutes TBA concentration increased to 99.29 where TBF concentration reduced to .025 wt.%.

## Claims

1. A process for removing tertiary butyl formate from impure tertiary butyl alcohol, characterized in that
said impure tertiary butyl alcohol is contacted in a treating zone with a styrene-divinyl benzene anion ion exchange resin,in liquid phase at a temperature within the range of 0 to 130°C and a pressure sufficient to keep the reactants in liquid phase and for a period of time sufficient to convert said tertiary butyl formate to formic acid and additional tertiary butyl alcohol.

2. A process as claimed in Claim 1, wherein the ion exchange resin is a styrene-divinyl benzene copolymer.

3. A continuous process for removing tertiary butyl formate from impure tertiary butyl alcohol containing from about 50 to about 5,000 ppm of tertiary butyl formate which comprises
continuously flowing said impure tertiary butyl alcohol through a bed of a styrene-divinyl benzene anion ion exchange resin in a treating zone in liquid phase under treating conditions including a temperature of 0 to 130°C and a pressure sufficient to keep the reactants in liquid phase at a weighted hourly space velocity of 2 to 5 kg of impure tertiary butyl alcohol per kg of bed per hour to convert said tertiary butyl formate to formic acid and additional tertiary butyl alcohol, and
continuously withdrawing a substantially tertiary butyl formate and formic acid-free treated tertiary butyl alcohol product from said treating zone.

## Patentansprüche

1. Ein Verfahren zur Entfernung von tert.-Butylformiat aus unreinem tert.-Butylalkohol, dadurch gekennzeichnet, daß
besagter unreine tert.-Butylalkohol in einer Behandlungszone mit einem Styrol-Divinylbenzol-Anionen-Ionenaustauschharz in flüssiger Phase bei einer Temperatur im Bereich von 0 bis 130°C und einem Druck, der ausreichend ist, um die Reaktanten in flüssiger Phase zu halten, und für einen Zeitraum, der ausreichend ist, um besagtes tert.-Butylformiat zu Ameisensäure und zusätzlichen tert.-Butylalkohol umzuwandeln, in Kontakt gebracht wird.

2. Ein Verfahren nach Anspruch 1, wobei das Ionenaustauschharz ein Styrol-Divinylbenzol-Copolymer ist.

3. Ein kontinuierliches Verfahren zur Entfernung von tert.-Butylformiat aus unreinem tert.-Butylalkohol, der von etwa 50 bis etwa 5.000 ppm tert.-Butylformiat enthält, welches umfaßt
kontinuierliches Fließenlassen von besagtem unreinen tert.-Butylalkohol durch ein Bett aus Styrol-Divinylbenzol-Anionen-Ionenaustauschharz in einer Behandlungszone in flüssiger Phase unter Behandlungsbedingungen, die eine Temperatur von 0 bis 130°C und einen Druck, der ausreichend ist, um die Reaktanten in flüssiger Phase zu halten, einschließen, bei einer gewichtsbezogenen stündlichen Raumgeschwindigkeit von 2 bis 5 kg unreinem tert.-Butylalkohol pro kg Bett pro Stunde, um besagtes tert.-Butylformiat zu Ameisensäure und zusätzlichem tert.-Butylalkohol umzuwandeln, und
kontinuierliches Abziehen eines im wesentlichen von tert.-Butylformiat und Ameisensäure freien, behandelten tert.-Butylalkoholproduktes aus besagter Behandlungszone.

## Revendications

1. Procédé pour éliminer du formiate de tert-butyle d'alcool tert-butylique impur, caractérisé en ce que :
l'alcool tert-butylique impur est mis en contact dans une zone de traitement avec une résine échangeuse d'ions anionique à base de styrène-divinyl benzène, en phase liquide a une température dans l'intervalle de 0 a 130°C, et sous une pression suffisante pour maintenir les réactifs en phase liquide et pendant une durée suffisante pour convertir le formiate de tert-butyle en acide formique et en alcool tert-butylique supplémentaire.

2. Procédé selon la revendication 1, dans lequel la résine échangeuse d'ions est un copolymère de styrène-divinyl benzène.

3. Procédé continu pour éliminer du formiate de tert-butyle d'alcool tert-butylique impur contenant d'environ 50 a environ 5000 ppm de formiate de tertbutyle, qui consiste :
a faire passer en continu ledit alcool tert-butylique impur dans un lit d'une résine échangeuse d'ions anionique a base de styrène-divinyl benzène dans une zone de traitement en phase liquide, dans des conditions de traitement incluant une température de 0 a 130°C et une pression suffisante pour maintenir les réactifs en phase liquide, avec une vitesse spatiale horaire pondérée de 2 a 5 kg d'alcool tert-butylique impur par kilo de lit par heure, pour convertir ledit formiate de tert-butyle en acide formique et en alcool tert-butylique supplémentaire, et
à extraire en continu un produit d'alcool tert-butylique traité substantiellement exempt de formiate de tert-butyle et d'acide formique, de ladite zone de traitement.
